# EUROPEAN PATENT APPLICATION

(11) **EP 1 911 449 A1**
(43) Date of publication of application: **16.04.2008**
(21) Application number: 06768421.7
(22) Date of filing: 28.07.2006
(51) Int. Cl.: A61K 31/245, A61K 45/00, A61P 1/18, A61P 35/00, A61P 35/04, A61P 43/00

(54) **ANTI-TUMOR AGENT COMPRISING 6'-AMIDINO-2'-NAPHTHYL4- GUANIDINOBENZOATE OR SALT THEREOF**

(30) Priority: 29.07.2005 JP 2005220590
(71) Applicant: TORII PHARMACEUTICAL CO., LTD., Tokyo 103 (JP); Uwagawa, Tadashi, Tokyo 158-0083 (JP)
(72) Inventor: TORII PHARMACEUTICAL CO., LTD., Tokyo 103 (JP); Uwagawa, Tadashi, Tokyo 158-0083 (JP)
(74) Representative: Weber, Thomas
(86) International application number: PCT/JP2006/315455
(87) International publication number: WO 2007/013696

(57) **Abstract**

It is an object of the present invention to provide novel antitumor agents which have a high therapeutic index while causing neither serious adverse effects nor drug resistance, both of which are the problems associated with existing antitumor agents. In particular, it is an object of the present invention to provide novel agents and therapies which are effective for pancreas cancer, for which no effective therapies and chemotherapeutic agents exist at this stage, and are also capable of inhibiting cancer metastasis and cancer filtration. The antitumor agents, more specifically the therapeutic agents for pancreas cancer and the cancer metastasis inhibitors, of the present invention contain as an active ingredient 6'-amidino-2'-naphthyl 4-guanidinobenzoate or a salt thereof or more specifically the mesylate thereof known as nafamostat mesilate (generic name; also referred to as "Futhan," "FUT," and "FUT175."). Moreover, in another mode of the present invention, the above mentioned FUT is administered in combination with existing anticancer/antitumor agents, particularly preferably in combination with gemcitabine hydrochloride.

## Description

### Technical Field

The present invention relates to antitumor agents, more specifically therapeutic agents for pancreas cancer and cancer metastasis inhibitors or cancer infiltration inhibitors, containing as an active ingredient 6'-amidino-2'-naphthyl 4-guanidinobenzoate or a salt thereof or more specifically the mesylate thereof known as nafamostat mesilate (generic name; also referred to as "Futhan," "FUT" and "FUT175;" abbreviated as "FUT" hereinafter.), and methods for treating tumors and cancers, wherein the methods are characterized in that the antitumor agents or inhibitors are administered. The present invention also relates to medicines containing FUT and existing anticancer/antitumor agents and methods for treating tumors and cancers, characterized in that the method includes administering the medicines.

### Background Art

Existing cancer therapy includes surgical therapy, radiation therapy, chemotherapy, hormonal therapy, immunotherapy and combinations thereof. Advances in surgical therapy as well as radiation therapy have allowed physicians to anticipate nearly perfect treatment for cancers of certain sites. However, the extent of surgical resection is limited. Chemotherapy may serve as the most effective treatment for patients whose cancer have relapsed after surgery or whose metastasis of cancers are not likely to be suppressed or who are not treatable by surgery. Chemotherapy may be also used a supplementary therapy to radiation therapy or to provide symptomatic relief.

Anticancer agents are categorized into 2 groups: "cytotoxic anticancer agents" and "molecular targeted therapeutic agents" based on their mechanisms of action, origins and the like. The "cytotoxic anticancer agents" are further categorized into antimetabolites, alkylating agents, anticancer antibiotics, microtubule inhibitors and the like. Alkylating agents and anticancer antibiotics, which are infallible with short duration of action above certain concentration thresholds, induce severe damage to normal cells. To overcome this problem, researchers are actively working on how to ensure medicines to more effectively reach cancer lesions. Recently, the discovery of targets with high specificity in cancers has led to the development of medicines which efficiently act on the targets (molecular targeted therapeutic agents). These agents have already started to be used by those involved in health care. Another problem is acquired resistance of cancer cells against chemotherapeutic agents, which shortens the duration of effects of chemotherapeutic agents. Therefore, the development of agents which show significant effects with fewer adverse drug reactions has been long-awaited.

Examples of chemotherapeutic agents currently being used include alkylating agents such as nitrogenmustard-N-oxide, cyclophosphamide, triethylenethiophosphoramide and sarcolysine, antimetabolites such as 5-fluorouracil, gemcitabine, cytarabine and methotrexate, anticancer antibiotics such as mitomycin C, actinomycin D, bleomycin, chromomycin A₃, daunomycin and doxorubicin, microtubule inhibitors such as vinorelbine, paclitaxel and docetaxel, and others such as irinotecan hydrochloride, cisplatin, carboplatin, etoposide, nedaplatin, mitoxantrone, L-asparaginase, procarbazine, colchicine derivatives and picibanil.

Any existing anticancer agent basically exerts a direct cytotoxic effect on cancer cells as its mechanism of action. However, investigations of thalidomide, which is no longer prescribed as a sleeping inducing agent due to its teratogenicity, have recently highlighted an entirely new concept in antitumor effect through pursuing the cause of teratogenicity. The main characteristic of this novel anticancer agent is to block neoangiogenesis instead of targeting cancer cells themselves. More specifically, it blocks oxygen and nutrition supply, which is necessary for cell proliferation, via inhibition of neoangiogenesis due to their antimitotic properties on vascular endothelial cells. This results in inhibition of cancer cell development as well as cancer metastasis. The agents, therefore, are expected to serve as ideal agents as they neither lead to resistance nor cause various serious adverse effects, which are specifically caused by conventional anticancer agents, due to lack of cytotoxic effects. The occurrence of neoangiogenesis in the adult is generally regulated so that it only takes place under certain diseased conditions such as wound healing, sexual cycle, cancer cells, rheumatism and ischemia. The regulation is accomplished through positive and negative regulators, intricately interacting with protease activities, activities of vascular endothelial cells, fibroblast activities, immune response, CXC chemokine regulation and the like.

For example, with regard to matrix metalloproteinases (MMPs) which promote neoangiogenesis and tissue inhibitors of MMP (TIMPs) which inhibit MMPs, elevated MMP expression has been reported in tumor cells. Therefore, MMP inhibitors which inhibit MMP expression are considered to be effective as anticancer agents. Examples of MMP inhibitors currently being developed include Marimastat (British Biotech, USA, Phase III trial in patients with nonsmall cell lung cancer, small cell lung cancer, breast cancer, prostate cancer and esophagus cancer), AG3340 (Agouron, USA, Phase III trial in patients with nonsmall cell lung cancer and hormone-refractory prostate cancer, Phase II trial in patients with neuroblastoma), COL-3 (Collagenex, USA, Phase I trial in patients with various kinds of solid cancers), Neovastat (Aeterna, Canada, Phase III trial in patients with nonsmall cell lung cancer and the like) and BMS-275291 (Bristol-Myers, USA, Phase I trial). Based on scientific knowledge that nuclear factor κB (throughout this specification, nuclear factor κB is abbreviated as NF-κB hereinafter) regulates MMP expression, inhibition of NF-κB activation is considered to be an effective way to inhibit elevated MMP expression in tumor cells.

Despite significant advances in anticancer agents and cancer therapies, pancreas cancer still has one of the worst prognoses. Even patients with operable pancreas cancer have a short survival time from 12 to 14 months after surgery. No agent has been proven to be effective enough for pancreas cancers at this stage. Therefore, the development of agents with novel mechanisms of action has been long-awaited. Under the circumstances, researchers recently attempt to enhance sensitivity to chemotherapeutic agents by use of an inhibitor of NF-κB which is known as a transcription regulator through a molecular biological approach. Drug resistance, which is one of the problems associated with conventional chemotherapeutic agents, is considered to be partly due to NF-κB activation triggered by the agents in cancer cells. Therefore, it is conceivable that one can prevent acquisition of drug resistance and sustain the effects of chemotherapeutic agents by inhibiting NF-κB which is activated by the existing chemotherapeutic agents.

NF-κB was first identified as a B cell-specific nuclear factor which binds to the immunoglobulin (Ig) κ chain gene enhancer. Subsequently, it has been revealed that NF-κB is involved in induction of gene expressions for various kinds of cytokines and receptors by binding to the promoter regions upstream of the genes and it plays an important role in immune response and onset of clinical conditions in inflammatory diseases. NF-κB is a protein complex composed of subunits belonging to the Rel protein family represented by p50 and p65 proteins and generally binds to IκB protein, which is a regulatory subunit, in the cytoplasm. However, upon a certain level of stimulation, IκB is phosphorylated by an IκB phosphorylating enzyme (IκB kinase complex: abbreviated as IKK hereinafter), allowing NF-κB to dissociate from the complex for activation. Activated NF-κB then translocates into the nucleus where it is involved in induction of gene expressions by binding to specific nucleotide sequences in the genomic DNA. Examples of genes whose expression is regulated by NF-κB include inflammatory cytokines such as IL-1, IL-6 and IL-8, and adhesion factors such as VCAM-1 and ICAM-1. NF-κB also regulates gene expression of its own inhibitor IκBα and thus forms a feedback loop.

Examples of diseases attributed to NF-κB include ischemic diseases, inflammatory diseases, autoimmune diseases, cancer metastasis, cancer infiltration and cachexia. Examples of ischemic diseases include ischemic organ diseases (ischemic heart diseases such as myocardial infarction, acute heart failure and chronic heart failure, ischemic brain diseases such as cerebral infarction, and ischemic lung diseases such as pulmonary infarction as an example), deterioration of prognosis after organ transplantation and organ surgery (deterioration of prognosis after cardiac transplantation, cardiac surgery, renal transplantation, renal surgery, liver transplantation, liver surgery, bone marrow transplantation, skin transplantation, corneal transplantation and lung transplantation as an example), reperfusion injury and restenosis after PTCA. Examples of inflammatory diseases include various inflammations such as kidney inflammation, hepatic inflammation and articular inflammation, acute renal failure, chronic renal failure and arteriosclerosis. Examples of autoimmune diseases include rheumatism, multiple sclerosis and Hashimoto's thyroiditis (Patent Document 1 for reference as an example).

Apart from inflammation reaction, NF-κB also plays an important role in cell proliferation and cell survival. It is said that impaired regulation of NF-κB causes many inflammatory diseases and cancers. For example, the present inventors reported that the p65 subunit of NF-κB is constantly activated in many human pancreas cancer tissues and human pancreas cancer cell lines, and also reported that hepatic metastasis or tumor formation of pancreas cancer cells can be suppressed by inactivating NF-κB.

Researchers also attempt to treat various diseases by inhibiting NF-κB. Proteasome inhibitors, IKK inhibitors and immunoregulatory agents such as thalidomide are known as NF-κB inhibitors. Moreover, it is reported, for example, that nonsteroidal agents such as aspirin and sodium salicylate have an inhibitory effect on NF-κB activation in high concentration (Non-Patent Document 1 for reference as an example), xanthine derivatives which contain purine in the structure have an inhibitory effect on NF-κB activation (Patent Document 2 for reference as an example), and dexamethasone which is a steroidal agent has an inhibitory effect on NF-κB activation via induction of IκB production (Non-Patent Document 2 for reference as an example). Some of the agents are under evaluation in clinical trials.

Agents containing as an active ingredient 6'-amidino-2'-naphthyl 4-guanidinobenzoate or a pharmacologically acceptable salt thereof or more specifically nafamostat mesilate (FUT) in the present invention have already been approved in Japan as being effective in improving acute symptoms associated with pancreatitis (acute pancreatitis, acute exacerbation of chronic pancreatitis, acute pancreatitis after surgery, acute pancreatitis after pancreatography and traumatic pancreatitis), improving disseminated intravascular coagulation (DIC), and preventing coagulation of blood in the perfusion system during extracorporeal circulation (hemodialysis and plasmapheresis) for patients with hemorrhagic lesion or hemorrhagic tendency. The safety of the agents has also been confirmed.

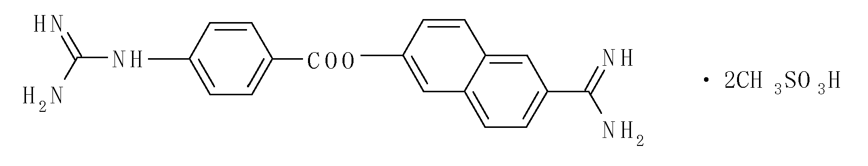

FUT also has an inhibitory activity on tryptase, which is a protease released from mast cells during degranulation. It is reported that FUT is effective in treating or preventing a disease selected from the group consisting of systemic anaphylaxis disease, aspirin-induced asthma, asthma, interstitial lung disease, interstitial cystitis, irritable bowel syndrome, allergic disease, atopic disease, skin fragility, hyperesthesia, pain, pruritus, gingivitis, edema, psoriasis, pulmonary fibrosis, articular inflammation, periodontal disease, blood coagulation disorder, renal interstitial fibrosis, adverse effects caused by X-ray contrast agent such as vascular hyperpermeability or pulmonary edema, and pollen allergy.

Furthermore, the present inventors found that FUT is effective as an immunomodulatory agent due to its inhibitory effect on inflammation reaction by inhibiting NF-κB activation in immunocompetent cells accumulating at local inflammatory sites.

As mentioned above, the anticancer agents which are currently in use have various problems, namely, such as acquired drug resistance via NF-κB activation in cancer cells, severe toxicity to normal cells and serious adverse effects. Therefore, the development of novel anticancer agents which neither induce drug resistance nor cause adverse effects has been long-awaited. The development of effective agents for pancreas cancer has also been anxiously awaited as it is often said that the disease currently has the worst prognosis of all cancer due to lack of effective therapies or anticancer agents at this stage.
Patent Document 1: Japanese Patent Laid-Open No. 2003/128559
Patent Document 2: Japanese Patent Laid-Open No. 9/227561
Non-Patent Document 1: Science, 265, 956 (1994)
Non-Patent Document 2: Science, 270, 283 (1995)

### Disclosure of the Invention

### Object of the Invention

The antitumor agents which are currently used as chemotherapeutic agents often cause serious adverse effects by affecting normal cells, more specifically rapidly dividing tissues such as marrow tissues and gonadal tissues while suppressing proliferation of malignancy by selectively inhibiting DNA and RNA synthesis in tumor cells or inhibiting tumor cell division. Adverse effects commonly caused by the agents include cardiac toxicity, nausea, vomiting, diarrhea, cytopenia, fever, acomia and hepatic damage. Therefore, the agents should be administered in a limited dosage, which is not effective enough to eradicate the tumors. This explains why the existing chemotherapeutic agents have not sufficiently demonstrated their effects under the current situation. Another problem associated with the existing chemotherapeutic agents is drug resistance acquired by tumor cells during the course of chemotherapy. Therefore, the development of novel agents whose activities are strong enough to overcome drug resistance and which have a high therapeutic index while exhibiting a low toxicity has been long-awaited. Furthermore, the development of novel medicines and therapies which can suppress cancer metastasis has been anxiously awaited. In particular, the development of effective therapies and novel agents for pancreas cancer has been anxiously awaited as the disease has the worst prognosis of all cancer due to lack of effective therapies or chemotherapeutic agents at this stage.

### Means for solving the Object

The present inventors have done earnest research to solve the above mentioned problems and concretely found that FUT, whose effectiveness and safety have already been confirmed for use as a therapeutic agent to pancreatitis, a therapeutic agent to diffuse intravascular coagulation (DIC), and a coagulation inhibitor for the perfused blood during extracorporeal circulation, is significantly effective as an agent with antitumor effect. Based on this scientific knowledge, the present inventors have achieved the present invention.

More specifically, the present inventors found that the agents containing as an active ingredient 6'-amidino-2'-naphthyl 4-guanidinobenzoate or a pharmacologically acceptable salt thereof or more specifically nafamostat mesilate (FUT) in the present invention exert their action on tumor cells and decrease the survival rate as well as induce apoptosis of tumor cells. The present inventors further conducted detailed research to find that the agents are novel agents, which inhibit proliferation of tumor cells through a mechanism whereby the agents inhibit NF-κB activation via inhibition on the phosphorylation of IκB-α by inhibiting IKK in tumor cells. Furthermore, the present inventors successfully confirmed that FUT has a significant effect on pancreas cancer, for which an effective therapeutic agent has currently been long-awaited. Above all, the present inventors successfully confirmed the effect of FUT *in vivo* through tests involving animals with transplanted human pancreas cancer cells. Based on this scientific knowledge, the present inventors have achieved the present invention.

In another mode of the present invention, the above mentioned FUT exerts a further significant effect when administered in combination with existing anticancer agents. The present inventors found that the combined administration shows no adverse effects such as weight reduction. Based on this scientific knowledge, the present inventors have achieved the present invention. More specifically, the existing anticancer agents have a problem of inducing drug resistance. In contrast, FUT has an inhibitory effect on the activation of NF-κB, a cause of drug resistance. Therefore, the coadministration of both makes it possible to further enhance the effects of the existing anticancer agents.

Surprisingly, the administration of FUT in combination with other agents, or more specifically in combination with gemcitabine, exerted a completely unexpected tumor suppressing effect as well as inhibitory effect on adverse effects. The concomitant effects were also confirmed in an animal study. The tumor suppressing effect and inhibitory effect on adverse effects of the combined administrations were particularly significant against pancreas cancer. In particular, the administration of FUT in combination with antitumor agents such as gemcitabine surprisingly and unexpectedly exerted a significant inhibitory effect on serious adverse effects such as weight reduction.

MMP, whose expression is elevated on the surface of cancer cells, is a molecule which is involved in swelling of cancer tissues as well as metastasis and infiltration of cancer cells. It was found that FUT has an inhibitory effect on MMP expression and thus is significantly effective not only as an antitumor agent but also as a metastasis inhibitor. In particular, the administration of FUT in combination with other antitumor agents such as gemcitabine is highly expected to be put into practical use not only from the standpoint of its pharmacological effect but also through its inhibitory effect on adverse effects such as weight reduction.

The present invention relates to antitumor agents containing as an active ingredient nafamostat mesilate. The present invention will now be described hereinafter.
(1) An antitumor agent, a cancer metastasis inhibitor, or a cancer infiltration inhibitor containing as an active ingredient 6'-amidino-2'-naphthyl 4-guanidinobenzoate represented by the following formula or a pharmacologically acceptable salt thereof. 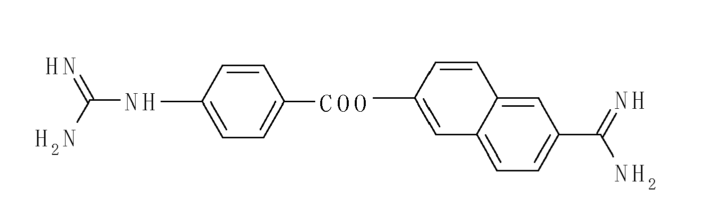
(2) The antitumor agent, the cancer metastasis inhibitor, or the cancer infiltration inhibitor according to above (1), wherein the salt is mesylate.
(3) The antitumor agent, the cancer metastasis inhibitor, or the cancer infiltration inhibitor according to either above (1) or (2), wherein the agent or inhibitor is an antitumor agent, a cancer metastasis inhibitor, or a cancer infiltration inhibitor for pancreas cancer respectively.
(4) An NF-κB inhibitor containing as an active ingredient 6'-amidino-2'-naphthyl 4-guanidinobenzoate or a pharmacologically acceptable salt thereof.
(5) The NF-κB inhibitor according to above (4), wherein the salt is mesylate.
(6) An IKK (IκB phosphorylating enzyme) inhibitor containing as an active ingredient 6'-amidino-2'-naphthyl 4-guanidinobenzoate or a pharmacologically acceptable salt thereof.
(7) The IKK (IκB phosphorylating enzyme) inhibitor according to above (6), wherein the salt is mesylate.
(8) A medicine containing an agent according to any one of above (1) to (7) in combination with other antitumor agents.
(9) The medicine according to above (8), wherein the other antitumor agents are one or more antitumor agents selected from the group consisting of alkylating agents, antimetabolites, antibiotics, plant alkaloids, platinum complex derivatives and hormones.
(10) The medicine according to either above (8) or (9), wherein the other antitumor agents are one or more antitumor agents selected from the group consisting of gemcitabine hydrochloride, nitrogen mustard N-oxide, cyclophosphamide, melphalan, carboquone, busulfan, nimastine hydrochloride, ranimastine, dacarbazine, fluorouracil, tegafur, cytarabine, ansaitabine hydrochloride, broxyuridine, doxifluuridine, mercaptopurine, thioinosin, methotrexate, mitomycin, bleomycin, daunorubicin hydrochloride, doxorubicin hydrochloride, pirarubicin hydrochloride, aclarubicin hydrochloride, neocarzinostatin, actinomycin D, vincristin hydrosulfate, vindesin hydrosulfate, vinblastin hydrosulfate, etoposide, tamoxifen citrate, procarbazine hydrochloride, mitobronitol, mitoxanthone hydrochloride, carboplatin and cisplatin.
(11) A method for treating or preventing cancers or tumors, characterized in that the method includes administering the antitumor agent, the cancer metastasis inhibitor, or the cancer infiltration inhibitor according to above (1) to a cancer patient or tumor patient.
(12) The method for treating or preventing cancers or tumors according to above (11), characterized in that the method includes administering the antitumor agent, the cancer metastasis inhibitor, or the cancer infiltration inhibitor according to above (1) in combination with other antitumor agents simultaneously or separately at any interval to a cancer patient or tumor patient.

### Effect of the Invention

In general, any anticancer agent commonly causes extreme adverse effects, which have been posing a major problem as they are agonizing for patients. In contrast, the agents containing as an active ingredient 6'-amidino-2'-naphthyl 4-guanidinobenzoate or a pharmacologically acceptable salt thereof or more specifically nafamostat mesilate (FUT) in the present invention have already been approved in Japan as being effective in improving acute symptoms associated with pancreatitis (acute pancreatitis, acute exacerbation of chronic pancreatitis, acute pancreatitis after surgery, acute pancreatitis after pancreatography and traumatic pancreatitis), improving disseminated intravascular coagulation (DIC), and preventing coagulation of blood in the perfusion system during extracorporeal circulation (hemodialysis and plasmapheresis) for patients with hemorrhagic lesion or hemorrhagic tendency. The safety of the agents has also been confirmed.

Therefore, it is expected that the development of anticancer agents containing as an active ingredient FUT, whose safety has been confirmed, and its application in therapy for pancreas cancer, for which no therapy has yet to be established, will have a profound effect on patients who are currently agonized from adverse effects caused by the existing anticancer agents.

Moreover, the agents not only have a direct antitumor effect but also have an inhibitory effect on cancer infiltration and cancer metastasis, which are part of the cancer growing process. Therefore, the agents are expected to exert effects as metastasis inhibitors after surgery.

Furthermore, the agents also have an inhibitory effect on drug resistance caused by the existing anticancer agents and antitumor agents. Therefore, the agents, when administered in combination with the existing anticancer agents and antitumor agents, can exert further enhanced anticancer/antitumor effects. In addition, FUT exerts significantly synergistic effects when administered in combination with the existing anticancer/antitumor agents. This has led to reduction in total dose of anticancer/antitumor agents needed. As a result, it has made it possible to reduce numerous adverse effects, which have been agonizing for patients. What is most remarkable is that these concomitant effects have been confirmed in an animal study conducted by the present inventors. In particular, the present inventors found that the agents had a significant inhibitory effect in animals with transplanted human pancreas cancer cells and showed no adverse effects such as weight reduction. These experimental facts indicate that the agents not only arouse a great hope on pancreas cancer, for which an effective therapy has been long-awaited but also have significant potential of clinical application.

### Brief Description of the Drawings

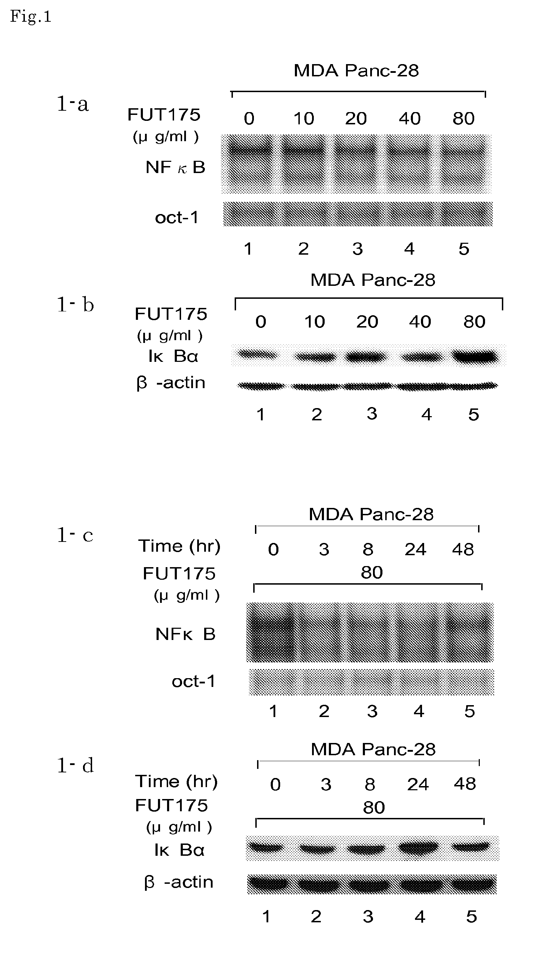

Figure 1 shows changes in NF-κB and IκBα by FUT treatment (Examples 1 and 2);
Figure 1-a and 1-c show the results of a gel shift assay detecting active NF-κB in the human pancreas cancer cell line MDAPanc-28 after being treated with various concentrations of FUT;
Figure 1-b and 1-d show the results of Western blot detecting IκB in the cytoplasmic fraction prepared from the human MDAPanc-28 after being treated with various concentrations of FUT;
Figure 2 shows changes in IKK activity and the level of phosphorylated IκBα, as well as apoptosis induction by FUT treatment (Examples 2, 3 and 4);

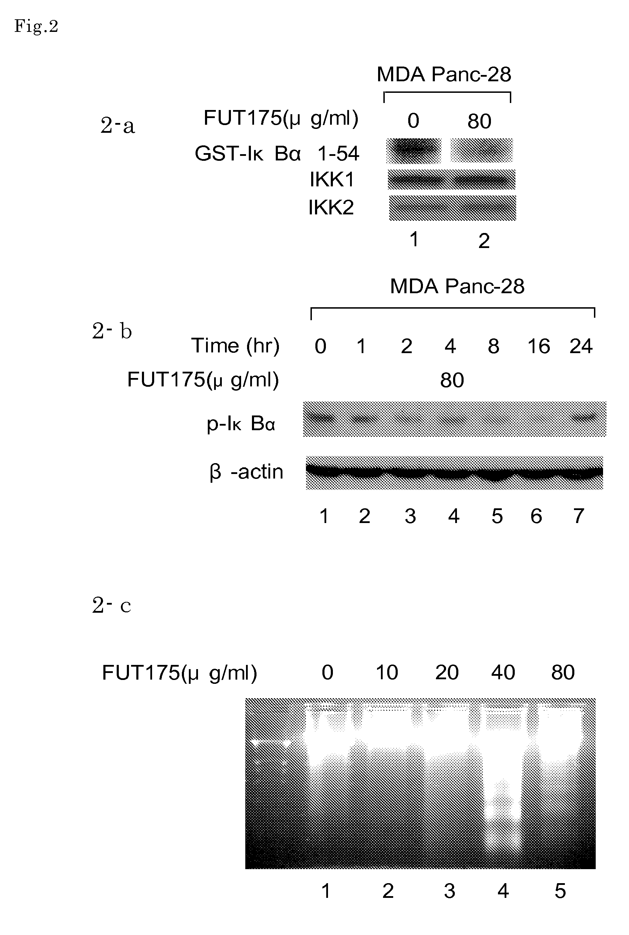

Figure 2-a shows the results of Western blot detecting the expression of IKK1 and IKK2 in the cytoplasmic fraction prepared from MDAPanc-28 before and after FUT treatment, and the detection results of enzyme activity (the level of phosphorylated IκBα) in each cytoplasmic fraction;
Figure 2-b shows the results of a timelapse detection of the level of phosphorylated IκBα in the cytoplasm of MDAPanc-28 after FUT treatment;
Figure 2-c shows the detection of DNA fragmentation after DNA extraction from MDAPanc-28 after being treated with various concentrations of FUT;

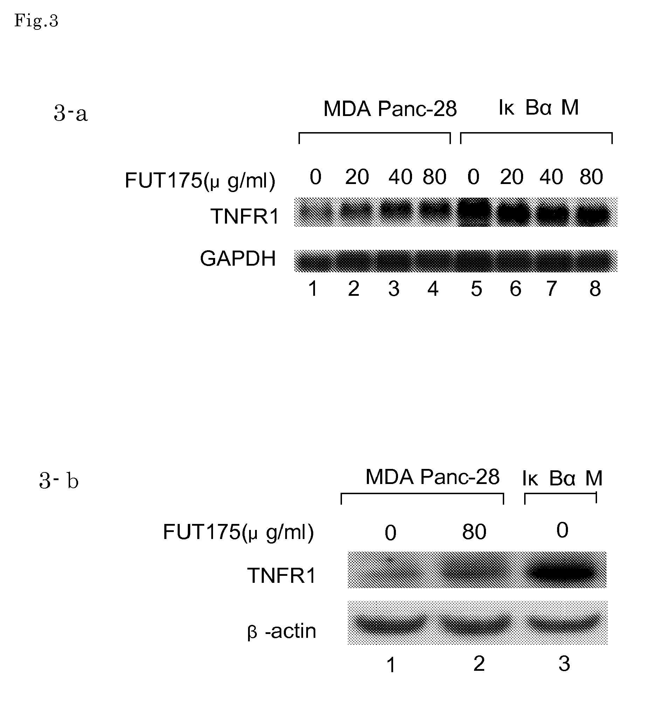

Figure 3 shows an elevation in TNFRI expression by FUT treatment (Example 5);
Figure 3-a and 3-b show changes in mRNA and protein expression of TNFRI in MDAPanc-28 cells after FUT treatment;

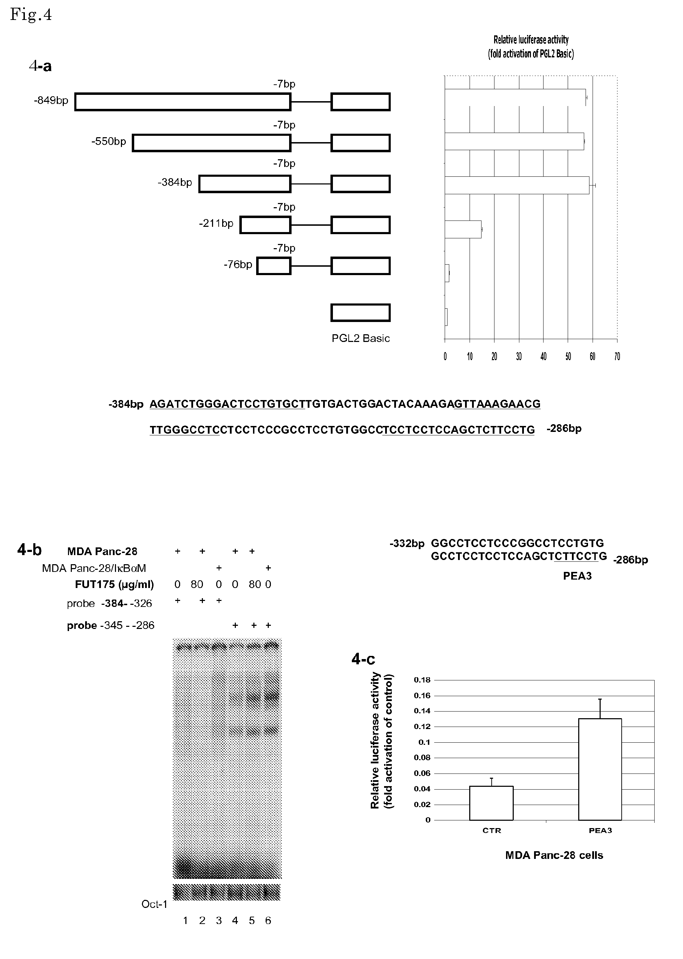

Figure 4 shows the results of examining the mechanism whereby TNFRI expression is elevated by FUT treatment (Example 6);
Figure 4-a shows the results of a reporter gene assay using cells transfected with vectors containing different lengths of TNFRI promoter. It was revealed that there was a binding site for a transcription factor inducing TNFRI expression between -384 bp and -211 bp;
Figure 4-b shows the results of a gel shift assay after PCR with 2 kinds of primers in order to identify the binding site for the transcription factor which induces TNFRI expression. It was revealed that there was a transcription factor binding site between -345 bp and - 286 bp;
Figure 4-c shows that the activation of TNFRI promoter was observed in cells transfected with PEA3 cDNA, but not in the control cells;

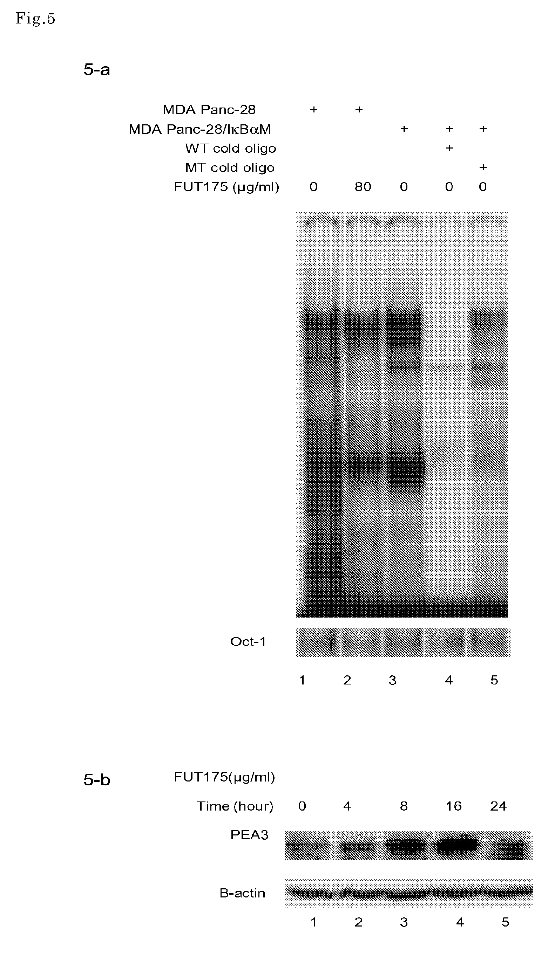

Figure 5 shows the results of examining the binding of PEA3 to the TNFRI promoter region (Example 6);
Figure 5-a shows the results of an inhibition experiment using a PEA3 probe and the partially mutated probe in order to confirm that PEA3 binds to the TNFRI promoter region;
Figure 5-b shows an elevation in PEA3 expression overtime in the human pancreas cancer cell line MDAPanc-28 by FUT treatment;

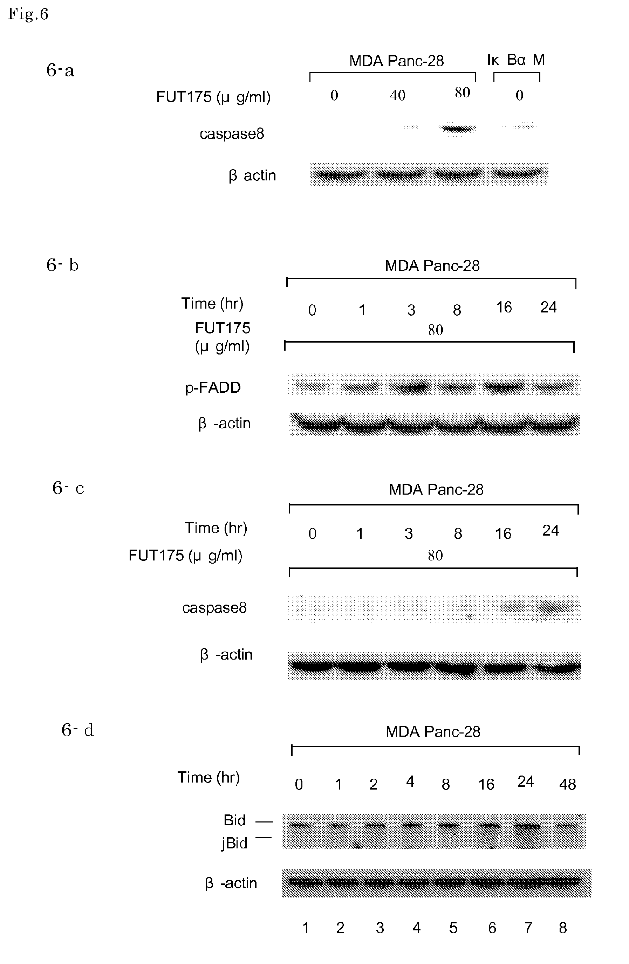

Figure 6 shows caspase 8 activation induced by FUT (Example 7);
Figure 6-a shows the results of Western blot detection with anti-caspase 8 antibody using the plasmatic compartment prepared from the human pancreas cancer cell line MDAPanc-28 after being treated with various concentrations of FUT for 24 hours;
Figures 6-b, 6-c and 6-d show the results of Western blot detecting changes in the expression of caspase 8, p-FADD, Bid and jBid overtime under a constant concentration of FUT (80 µg/ml) at short intervals from 0 to 24 hours of treatment;

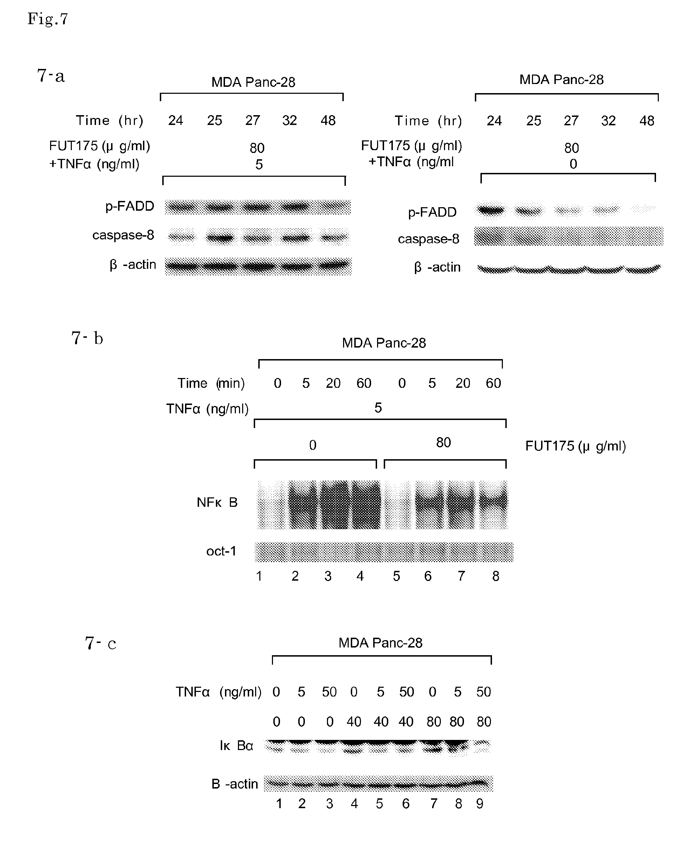

Figure 7 shows the concomitant effects of FUT and TNF-α (Examples 8 and 9); the results of Western blot detecting the expression of phosphorylated FADD, caspase 8, NF-κB and IκBα in the cytoplasmic fraction prepared from the human pancreas cancer cell line MDAPanc-28 after the following treatment;
Figure 7-a shows the results for cells stimulated by TNF-α (5 ng/ml) for 24 hours after pretreatment with FUT (80 µg/ml) ;
Figure 7-b shows that FUT treatment inhibits NF-κB activated by TNF-α;
Figure 7-c shows IκBα expression;

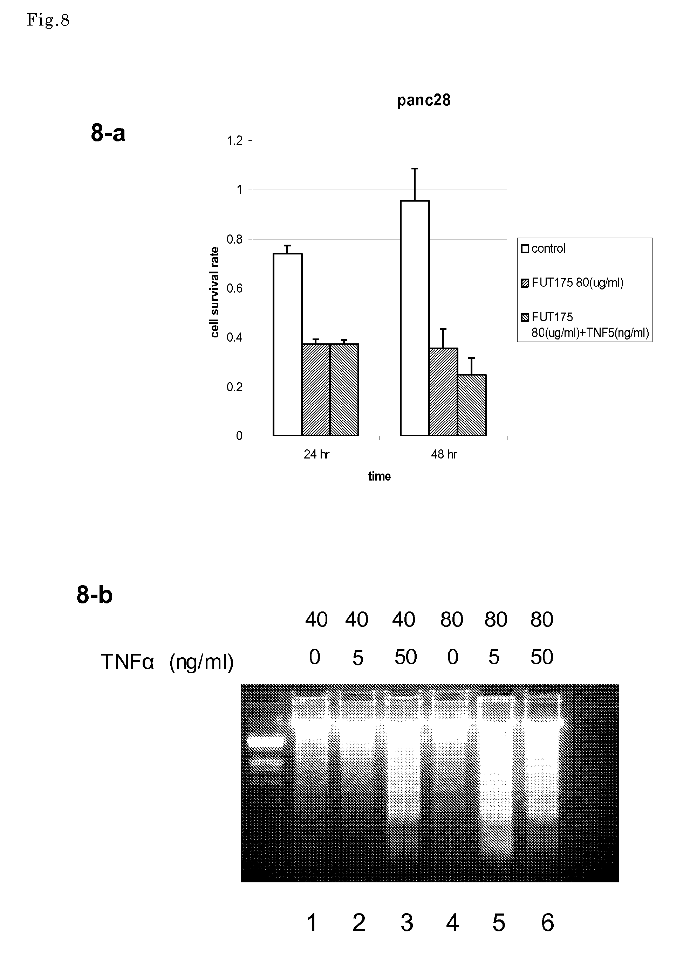

Figure 8 shows the concomitant effects of FUT and TNF-α (Example 9). The human pancreas cancer cell line MDAPanc-28 was first treated with TNF-α for 24 hours and then cultured with various concentrations of FUT being added for additional 24 hours;
Figure 8-a shows the results of cell survival rate calculation based on a MTT assay using the cells cultured as described above;
Figure 8-b shows the results of a DNA fragmentation assay after DNA extraction;

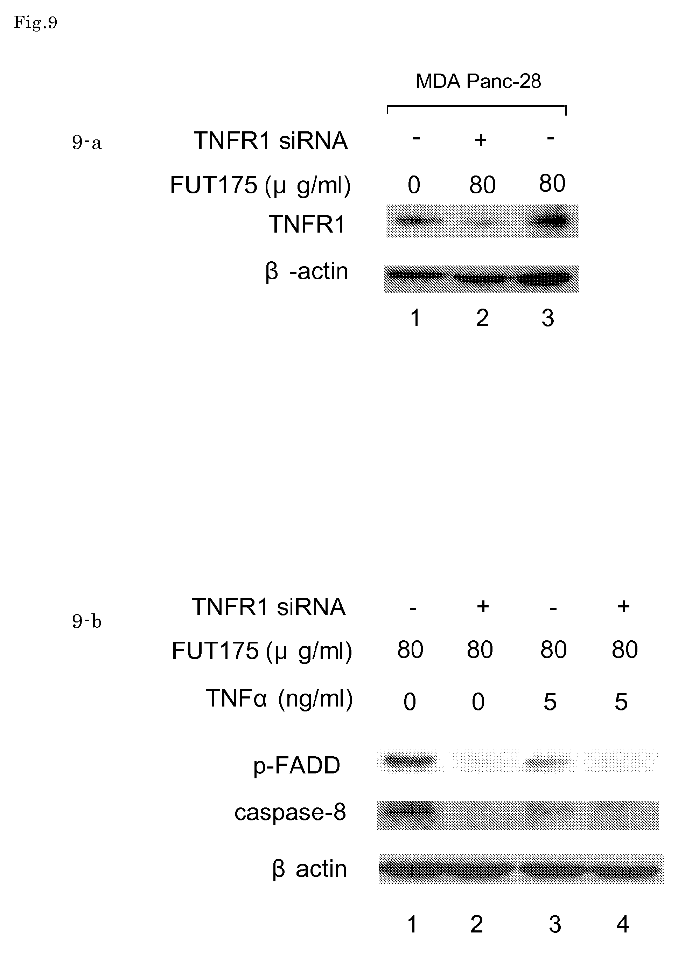

Figure 9 shows the results of examining the mechanism whereby FUT activates caspase 8 (Example 10). It shows the results of examining FADD phosphorylation and caspase 8 activation after FUT treatment of cells in which TNFRI expression is downregulated using siRNA (small interfering RNA);

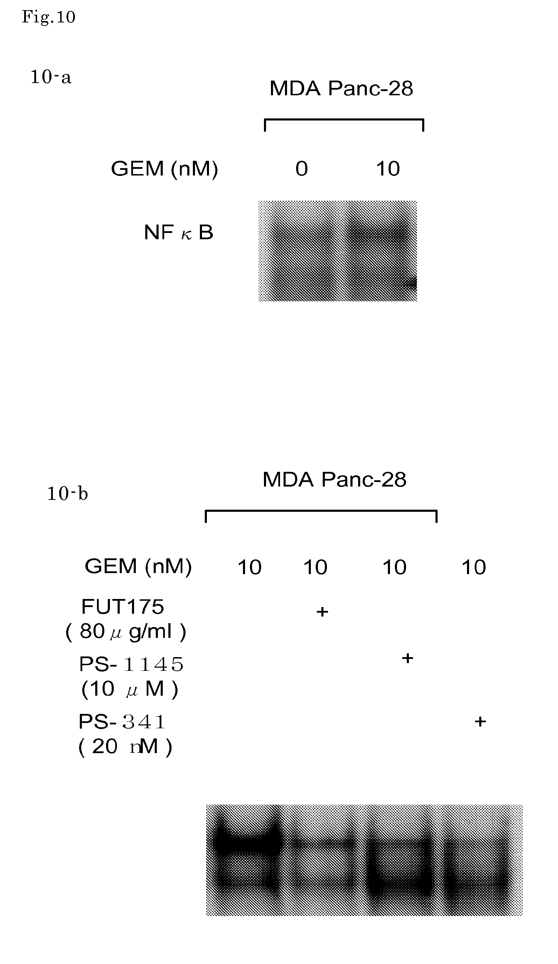

Figure 10 shows that FUT treatment inhibits the antitumor agent gemcitabine-induced activation of NF-κB (Example 11). It shows the results of a gel shift assay examining the presence of NF-κB activation in the human pancreas cancer cell line MDAPanc-28 after being treated with gemcitabine for 12 hours;
Figure 10-a shows the results of further examining the concomitant effects of gemcitabine and FUT;
Figure 10-b shows the results of a gel shift assay using the human pancreas cancer cell line MDAPanc-28 treated with gemcitabine for 3 hours after pretreatment with FUT, PS1145 or PS341 for 3 hours (Example 11);

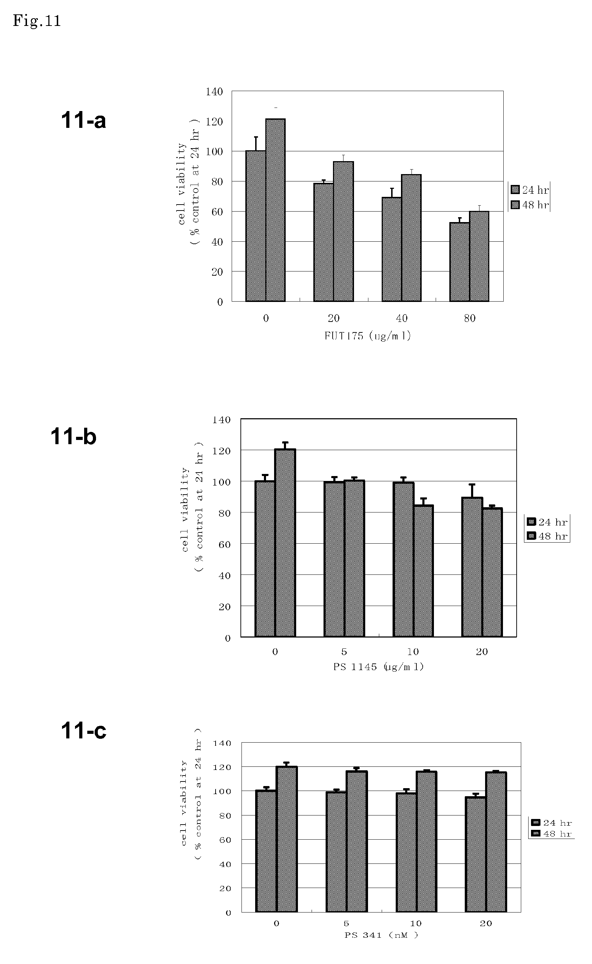

Figure 11 shows the results of examining the effect of FUT treatment on the living cell ratio in the human pancreas cancer cell line MDAPanc-28 (Example 12). The effect of FUT, PS1145 or PS341 on the living cell ratio in the human pancreas cancer cell line MDAPanc-28 was singularly examined. The figure shows the results of a MTT assay using the human pancreas cancer cell line MDAPanc-28 after being treated with each agent for 24 hours and 48 hours;

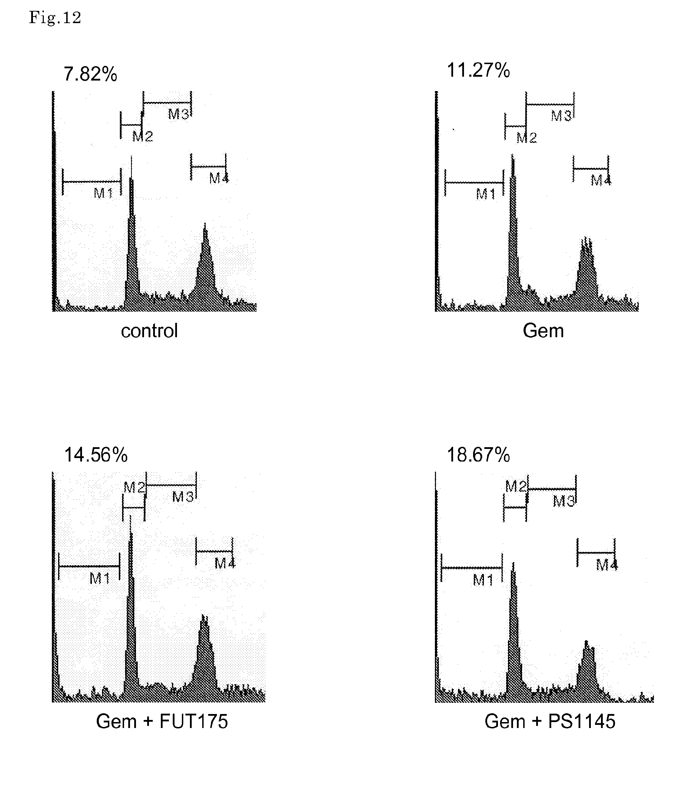

Figure 12 shows the results of examining the concomitant effect of gemcitabine and NF-κB on apoptosis in the human pancreas cancer cell line MDAPanc-28 (Example 13). Appoptosis-indusing cell ratio was calculated using the human pancreas cancer cell line MDAPanc-28 treated with gemcitabine for 48 hours after pretreatment with FUT, PS1145 or PS341 for 3 hours. FACScan measurements were used to confirm the occurrence of apoptosis;

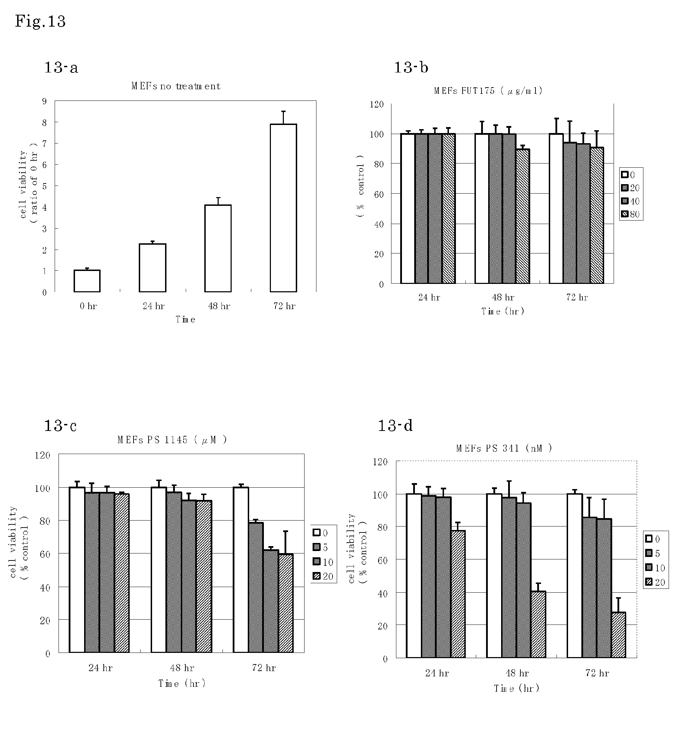

Figure 13 shows the effect of FUT treatment on normal cells (Example 14). It shows the results of examining the effect of FUT treatment on murine fiber cells;

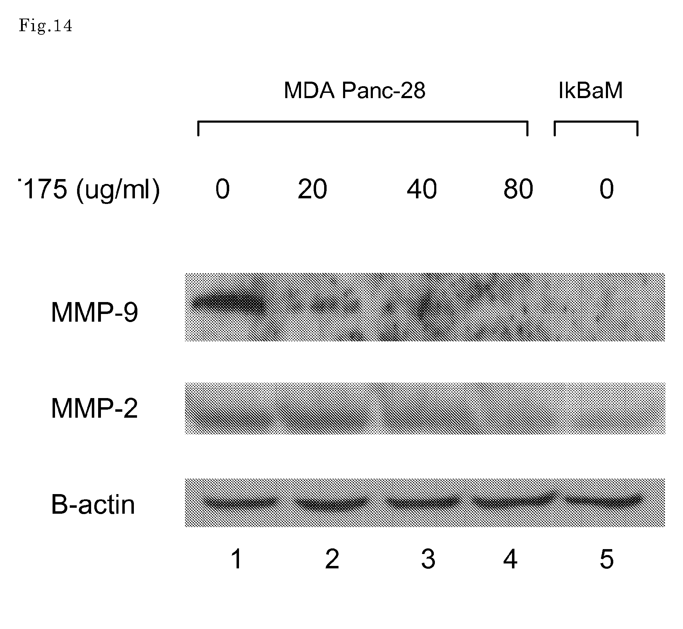

Figure 14 shows inhibition of MMP-2 and MMP-9 expression by FUT treatment (Example 15). It shows the results of Western blot examining MMP-2 and MMP-9 expression in the cytoplasmic fraction prepared from the human pancreas cancer cell line MDAPanc-28 after being treated with either 0 µg/ml or 80 µg/ml of FUT;

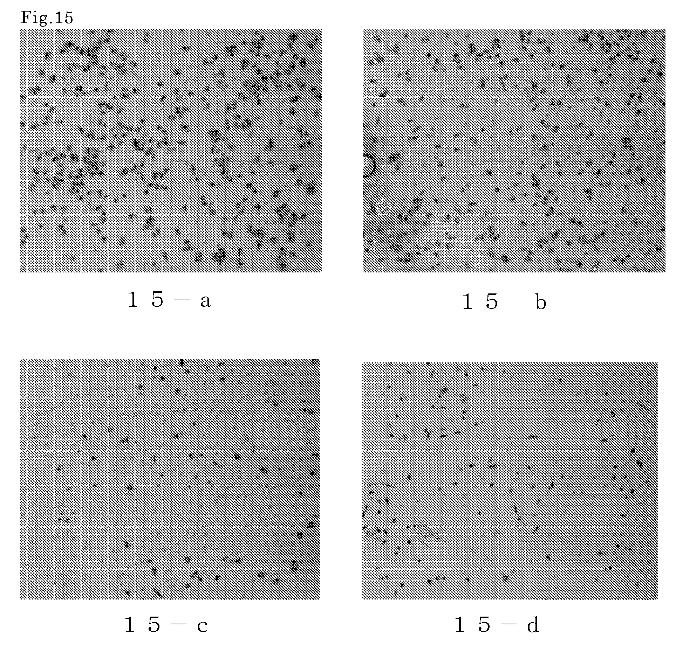

Figure 15 shows the results of examining an inhibitory effect of FUT treatment on infiltration in the human pancreas cancer cell line MDAPanc-28 (Example 16). Cells treated with 80 µg/ml of FUT for 24 hours and untreated cells were respectively inoculated to a matrigel-coated chamber at a concentration of 20000 cells per well for a 22-hour incubation at 37°C either in the presence or absence of 80 µg/ml of FUT. The numbers of infiltrated cells were then counted under the microscope after Wright-Giemsa staining. The results are shown in Figure 15;
The results for untreated cells (control) are shown in Figure 15-a, cells treated with FUT175 (80 µg/ml) for 22 hours are in Figure 15-b, cells pretreated with FUT175 (80 µg/ml) for 24 hours are in Figure 15-c, and cells treated with FUT175 (80 µg/ml) for 22 hours after pretreatment with FUT175 (80 µg/ml) for 24 hours are in Figure 15-d;

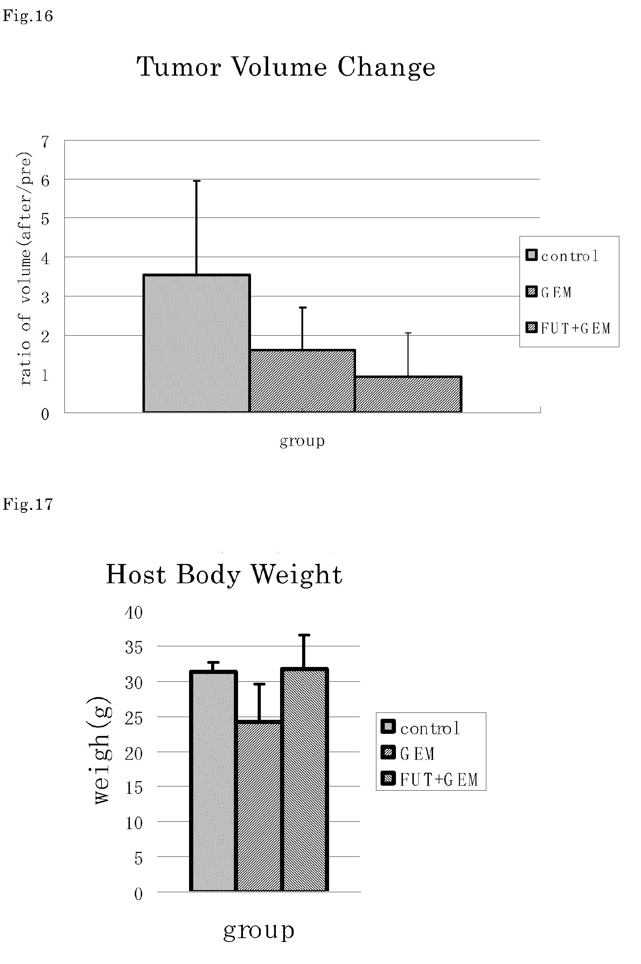

Figure 16 shows the results of measuring tumor volume for each group prepared as described hereinafter. Samples were divided into 3 groups so that each group had a similar average tumor radius 6 weeks after subcutaneous transplantation of the human pancreas cancer cell line Panc-1 (5 × 10⁶ cells). Each group was then subjected to the following treatment for 6 weeks respectively: the control group received no treatment (n=4); the GEM group (gemcitabine administered group) was administered with gemcitabine (100 mg/kg, once a week, i.p.) (n=4); and the FUT group was administered with FUT-175 (30 mg/kg, three times a week, i.p.) and gemcitabine (100 mg/kg, once a week, i.p.) (n=4) (Example 17);
Figure 17 shows the results of measuring body weight in mice after the treatments described in Figure 16 above (Example 17);

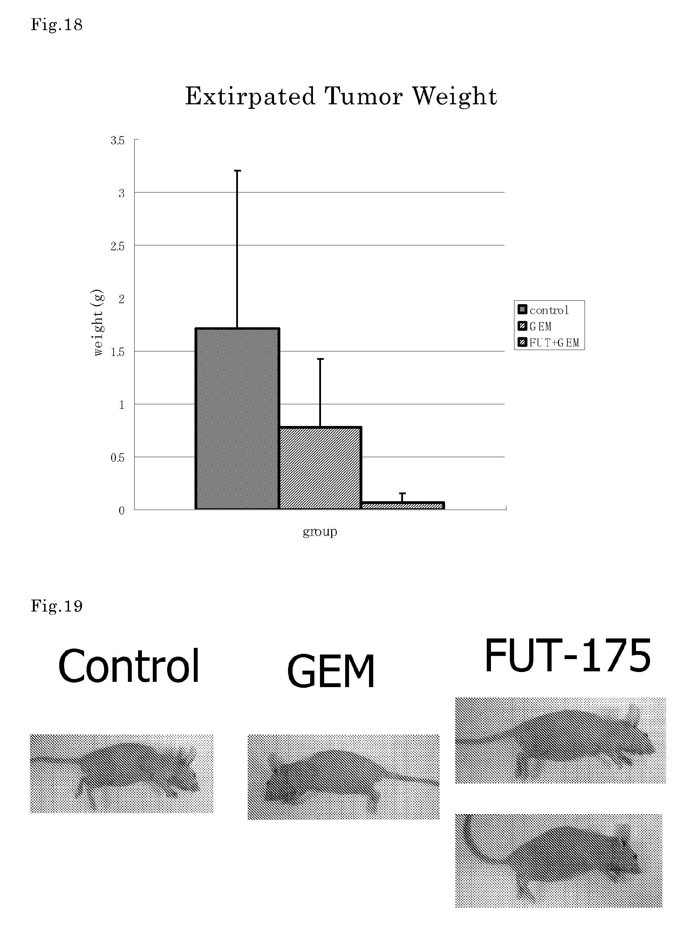

Figure 18 shows the results of measuring extirpated tumor weight in mice after the treatments described in Figure 16 above (Example 17); and
Figure 19 shows a set of pictures of mice taken after the treatments described in Figure 16 above.

### Best Mode for Carrying Out the Invention

As used herein, "Nafamostat mesilate (FUT)" represents 6-amidino-2-naphthyl p-guanidinobenzoate dimethanesulfonate represented by the following formula as described below.

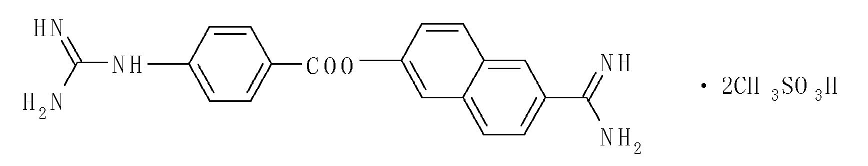

Nafamostat mesilate (FUT) has already been approved in Japan and its indicatios are:
1) improving acute symptoms associated with pancreatitis (acute pancreatitis, acute exacerbation of chronic pancreatitis, acute pancreatitis after surgery, acute pancreatitis after pancreatography and traumatic pancreatitis);
2) disseminated intravascular coagulation (DIC); and
3) preventing coagulation of blood in the perfusion system during extracorporeal circulation (hemodialysis and plasmapheresis) for patients with hemorrhagic lesion or hemorrhagic tendency.

The dosage of nafamostat mesilate (FUT) in the present invention varies in accordance with subjects being administered, routes of administration, target diseases, the degree of symptoms and the like. For example, the recommended dose for oral administration is preferably in the range of 2.8 mg/kg to 9.6 mg/kg per day. The recommended dose for parenteral administration varies in accordance with the forms of administration (such as injection formulations, external preparations and suppositories) and is preferably in the range of 1.4 mg/kg to 4.8 mg/kg per day.

The forms of the agents in the present invention include but are not limited to solid compositions, liquid compositions and other compositions for oral administration, and injection formulations, external preparations, suppositories, percutaneously absorbed preparations, inhalants and the like for parenteral administration. The solid compositions for oral administration contain tablets, pills, capsules, powders and granules. The capsules contain hard capsules and soft capsules. Injection formulations for parenteral administration are preferred.

In the solid compositions, the active substance composed of FUT is mixed with at least one inactive diluting agent such as lactose, mannitol, mannite, glucose, hydroxypropylcellulose, microcrystal cellulose, starch, polyvinylpyrrolidone and magnesium aluminometasilicate. The compositions may contain additives other than the inactive diluting agent, for example, lubricant agents such as magnesium stearate, disintegrating agents such as calcium cellulose glycolate, and solubilizing agents such as glutamic acid and aspartic acid according to the method generally known. The tablets and pills, if desired, may be coated with a film consisting of stomach-soluble or intestine-soluble materials such as sucrose, gelatine, hydroxypropylcellulose and hydroxypropylmethylcellulosephthalate, or may be coated with 2 or more films. Moreover, the capsules contain capsules consisting of absorbable materials such as gelatine.

The liquid compositions for oral administration contain pharmaceutically acceptable emulsion, solutions, syrups, elixirs and the like. In the liquid compositions, the active substance composed of FUT is dissolved in a generally used inactive diluting agent (such as purified water and ethanol). The liquid compositions may contain additives other than the inactive diluting agent, for example, auxiliary substances such as a moistening agent and a suspending agent, sweetening agents, flavoring agents and antiseptic agents.

The other compositions for oral administration contain sprays which contain the active substance composed of FUT and are prescribed under the conventional methods. Such compositions may contain additives other than the inactive diluting agent, for example, stabilizing agents which give isotonicity to a fixing agent such as sodium acid sulfite, and isotonic agents such as sodium chloride, sodium citrate and citric acid.

In relation to the solutions for parenteral administration, for example, injection formulations in the present invention contain sterile aqueous or nonaqueous solutions, suspension agents and opalizers. The aqueous solutions and suspension agents contain, for example, injectable distillated water and saline. The nonaqueous solutions and suspension agents contain, for example, vegetable oils such as propylene glycol, polyethylene glycol and olive oil, alcohols such as ethanol, and polysorbate 80 (registered trademark). Such compositions may further contain auxiliary substances such as preservatives, moistening agents, emulsifying agents, dispersing agents, stabilizing agents and solubilizing agents (for example, glutamic acid and aspartic acid). These are sterilized by filtration with a bacterial retention filter, dispensing pesticide or irradiation. They may also be produced in the form of sterile solid compositions, which can be dissolved in sterilized or sterile injectable distilled water or some other sterilized or sterile solvents before use.

The other compositions for parenteral administration contain enema preparations, external solutions, ointments, endermic liniments and suppositories which contain the active substance composed of nafamostat mesilate (FUT) and are prescribed according to the method generally known.

To be used as inhalations and aerosol preparations, an aerosol container is filled with the active substance in the form of solution, suspension or micropowder in combination with gas or liquid propellant and, if desired, conventional auxiliary substances such as a moistening agent and a dispersing agent. The compounds of the present invention may also be formulated as nonpressurized preparations such as a nebulizer and an atomizer.

Existing anticancer/antitumor agents to be administered in combination are not limited to but may be, for example, one or more antitumor agents selected from the group consisting of gemcitabine hydrochloride, nitrogen mustard N-oxide, cyclophosphamide, melphalan, carboquone, busulfan, nimastine hydrochloride, ranimastine, dacarbazine, fluorouracil, tegafur, cytarabine, ansaitabine hydrochloride, broxyuridine, doxifluuridine, mercaptopurine, thioinosin, methotrexate, mitomycin, bleomycin, daunorubicin hydrochloride, doxorubicin hydrochloride, pirarubicin hydrochloride, aclarubicin hydrochloride, neocarzinostatin, actinomycin D, vincristin hydrosulfate, vindesin hydrosulfate, vinblastin hydrosulfate, etoposide, tamoxifen citrate, procarbazine hydrochloride, mitobronitol, mitoxanthone hydrochloride, carboplatin and cisplatin. Gemcitabine hydrochloride is particularly preferred.

The preferred dosages of existing anticancer/antitumor agents for to be administered in combination are determined in accordance with the dosages regulated or recommended by FDA and the Ministry of Health, Labour and Welfare. However, as described above, FUT enhances the effects of the existing agents when administered in combination and thus the existing agents can exert sufficient effects at fewer dosages than the above described regular dosages. Therefore, the dosages of the existing anticancer/antitumor agents are more preferably in the range of 0.3 times to 1 time of the dosages regulated or recommended by FDA and the Ministry of Health, Labour and Welfare.

Moreover, the mode of administration of the existing anticancer/antitumor agents will be determined in accordance with the methods regulated or recommended by FDA and the Ministry of Health, Labour and Welfare. Regarding the timing for administering the existing anticancer/antitumor agents in combination with FUT, both agents may be simultaneously administered to patents or, alternatively, FUT may be administered prior to or after the existing anticancer/antitumor agents.

While the present invention will now be described in connection with certain examples more specifically hereinafter, it is understood that it is an exemplification and is not intended to limit the present invention to these particular examples.

### Example 1

### NF-κB inhibition by FUT175;

The human pancreas cancer cell line MDAPanc-28 was treated with various concentrations of FUT to examine the effect on NF-κB activation. NF-κB is constantly activated in the MDAPanc-28 cell line. A gel shift assay with a κB probe was performed using the nuclear extracts from cells treated with various concentrations of FUT to detect NF-κB which was activated and translocated into the nucleus. More specifically, 10 µg of nuclear extracts were reacted with 1 µg of poly (dI-dC) (Pharmacia, Picataway, N.J) in binding buffer (75 mM NaCl, 15 mM Tris-HCl, pH 7.5, 1.5 mM dithiothreitol, 25% glycerol and 20 mg/ml bovine serum albumin) for 30 minutes at 4°C. ³²P-labeled oligonucleotide probes (SEQ ID Nos: 1 to 4) were then added for reaction for 20 minutes at room temperature and electrophoresis was performed (4% polyacrylamide gel, 0.25 × TBE; 22.5 mM Tris, 22.5 mM borate and 500 µM EDTA, pH 8.0). After drying at 80°C for 1 hour, the gel was exposed to Kodak film (Eastman Kodak Co., Rochester, N.Y.) at -80°C for detection. Oct-1 was used as a positive control.

The results are shown in Figures 1-a and 1-c. FUT inhibited NF-κB activation in a concentration-dependent manner. It was also indicated that NF-κB activation was inhibited overtime up to 24 hours after addition of FUT and then was restored to a steady-state level at 48 hours.

### Example 2

### Reduction of phosphorylated IκBα by FUT;

The human pancreas cancer cell line MDAPanc-28 was treated with various concentrations of FUT to examine the effect on IκBα. Cytoplasm extracts were extracted from cells treated with various concentrations of FUT according to the method generally known and Western blot was performed to detect each protein. More specifically, each lane was loaded with 50 µg of protein to perform electrophoresis. The gel was then transferred to a PVDF membrane (Osmonics). After blocking with PBS containing 5% nonfat milk for 2 hours at room temperature, primary antibody (anti-IκBα antibody) diluted with PBS containing 0.1% nonfat milk was added for reaction at 4°C overnight. After rinsing with PBS, the PVDF membrane was then reacted with secondary antibody solution diluted with PBS containing 0.1% nonfat milk for 2 hours at room temperature. Proteins were detected by using Lumi-Light Western Blotting Substrate.

The results are shown in Figure 1-b, 1-d and 2-b. Figure 1-b shows that the level of IκBα in the cytoplasm is increased by FUT in a concentration-dependent manner. This indicates that the level of IκBα bound to inactive NF-κB increases in a FUT concentration-dependent manner in the cytoplasm. Figure 1-d shows that the level of IκBα in the cytoplasm increases along the progression of NF-κB inactivation up to 24 hours after addition of FUT and then is restored to a steady-state level after 48 hours. This result correlates with NF-κB activation described in Example 1. Experiments shown in Figure 2-b use an antibody which only recognizes phosphorylated IκBα as primary antibody. The figure shows that the level of phosphorylated IκBα in the cytoplasm declines overtime by adding FUT, indicating that FUT inhibits NF-κB activation.

### Example 3

### Changes in IKK activity by FUT;

After FUT treatment, the human pancreas cancer cell line MDAPanc-28 was rinsed with ice-cold PBS to prepare 800 µg of cytoplasmic fraction. After adding glutathione sepharose beads (Amersham Pharmacia Biotech), the sample was invertedly mixed at 4°C for 3 hours to remove nonspecific absorption fractions. 4 µg of anti-IKK1/2 antibody (Santa Cruz Biotechnology) were added to the supernatant for reaction at 4°C overnight. After adding glutathione sepharose beads, the sample was further mixed at 4°C for 3 hours. The beads containing bound immunocomplex were rinsed twice with PBS and then subjected to immunoprecipitation and measurements of enzyme activity.

To conduct the measurements of enzyme activity, the above mentioned beads were rinsed with kinase buffer (25 mM HEPES containing 150 mM NaCl, 25 mM beta-glycerophosphate and 10 mM MgCl₂) to perform enzyme reaction. 5 uCi of ³²P-labeled γ-ATP and 1 µg of the substrate GST-IκBα were reacted in the kinase buffer for 30 minutes at 37°C. The reaction was terminated by adding SDS sample buffer. The sample was analyzed by SDS-PAGE followed by autoradiography for detection.

The results are shown in Figure 2-a. It is indicated that FUT treatment reduces the enzyme activities of IKK1 and IKK2 as well as the level of IκBα to be phosphorylated, although the expression levels of IKK1 and IKK2 remain intact.

### Example 4

### Apoptosis induction in pancreas cancer cells by FUT;

After FUT treatment, the human pancreas cancer cell line MDAPanc-28 was incubated in DNA extraction buffer (10 mM Tris, pH 8.0 containing 0.1 mM EDTA, 0.5% SDS and 20 µg/ml RNase) for 1 hour at 37°C. After adding 1000 U/ml of protease K, the sample was further incubated for 5 hours at 50°C. After extracting DNA with phenol/chloroform/isoamyl alcohol, isopropanol and ammonium acetate were added to the collected watersoluble fractions in order to collect precipitates. The precipitates were then rinsed with 70% ethanol and dissolved in Tris-EDTA buffer. Electrophoresis was performed on a 2% agarose gel and DNA bands were confirmed by using ethidium bromide.

The results are shown in Figure 2-c. It was indicated that DNAs were fragmented in the MDAPanc-28 cells treated with FUT at a concentration of 40 µg/ml or higher, suggesting that FUT had induced apoptosis in the cells.

### Example 5

### Elevated expression of TNF receptor type 1 (abbreviated as TNFRI hereinafter) by FUT;

To reveal the mechanism of action whereby apoptosis is induced by FUT in cancer cells, the present inventors confirmed changes in TNFRI expression induced by FUT treatment in the human pancreas cancer cell line MDAPanc-28.

A cDNA probe for hybridization was constructed by the following method. After FUT treatment, RNA was extracted from the human pancreas cancer cell line MDAPanc-28 by using TRIZOL Reagent (Life Technologies, Inc.) 15 µg of RNA was subjected to electrophoresis on a 1% denatured formaldehyde agarose gel and transferred to a nylon membrane. RT-PCR was performed in order to construct a cDNA probe to be used for the detection of TNFRI mRNA. With this RT-PCR, primers represented by SEQ ID Nos: 5 and 6 were used. The obtained cDNA was inserted into the pCR2.1-TOPO vector. The nucleotide sequence of the obtained cDNA was found to match with the sequence registered in Gene Bank. The obtained cDNA probe was labeled with α-³²P dCTP by using the random labeling kit (Roche) and used for hybridization.

The expression of TNFRI mRNA was confirmed by Northern blot method with the above mentioned probe according to the method generally known. As shown in Figure 3-a, the level of TNFRI mRNA expression increased in a FUT concentration-dependent manner. Also, TNFRI expression on the cell surface was detected by Western blot method. More specifically, the experiment was performed by the method similar to the method described in Example 2. Mouse monoclonal anti-TNFR1 (H-5; Santa Cruz Biotechnology) was used for detection. As shown in Figure 3-b, the results indicated that FUT treatment increased TNFRI expression on the cell surface.

Combining the results obtained in Example 1, active NF-κB and TNFRI regulate reciprocal expressions though feedback regulation.

### Example 6

### Activation of the transcription factor PEA3 by FUT;

In order to identify the transcription factor involved in feedback regulation of NF-κB and TNFRI, vectors containing different lengths of TNFRI promoter were constructed to perform a reporter gene assay. The results revealed that there was a binding site for a transcription factor which induces TNFRI expression between -384 bp and -211 bp.

Moreover, in order to identify the binding site for the transcription factor which induces TNFRI expression, PCR was performed with 2 pairs of primers (SEQ ID Nos: 7 and 8 as well as SEQ ID Nos: 8 and 9). As a result, 2 kinds of probes were constructed. One probe recognizes the first half of the sequence between -384 bp and -286 bp and the other probe recognizes the second half of the sequence. A gel shift assay was performed with these probes by the method similar to Example 1. The results revealed that there was a transcription factor binding site between -345 bp and -285 bp. Analysis using the search software IFTI-MIRAGE identified the transcription factor binding to the sequence as PEA3 (Figure 4).

The human pancreas cancer cell line MDAPanc-28 was transfected with a vector containing luciferase gene ligated to the TNFRI promoter region as well as PEA3 cDNA, and was subjected to a reporter gene assay. The activation of TNFRI promoter was observed in cells transfected with PEA3 cDNA. However, this activation was not observed in control cells which were not transfected with PEA3 cDNA.

In order to confirm that PEA3 binds to the TNFRI promoter region, an inhibition experiment was performed with a PEA3 probe and the partially mutated probe. In cells with constantly inactivated NF-κB (MDAPanc-28/IκBα), TNFRI expression was high and labeled PEA3 probe was bound to the TNFRI promoter region. In contrast, comparison of the inhibitory activities of the 2 above mentioned non-labeled probes used for transfection revealed that cells transfected with the non-labeled PEA3 probe showed binding inhibition against the labeled PEA3 probe, whereas cells with the non-labeled mutated probe did not show any binding inhibition (Figure 5-a). It was also indicated that FUT treatment increased PEA3 expression overtime in the human pancreas cancer cell line MDAPanc-28 (Figure 5-b).

### Example 7

### Activation of caspase 8 by FUT;

The present inventors examined whether apoptosis signaling cascade is triggered with the elevation of TNFRI expression induced by FUT treatment, using caspase 8 as an indicator. Cytoplasmic fraction was prepared from the human pancreas cancer cell line MDAPanc-28 treated with various concentrations of FUT for 24 hours. Western blot was performed and subjected to the detection with anti-caspase 8 antibody (Santa Cruz Biotechnology) (Figure 6-a). Western blot was performed to examine temporal changes in the expression of caspase 8, p-FADD, Bid and jBid under a constant concentration of FUT (80 µg/ml) at short intervals from 0 to 24 hours of treatment (Figures 6-b, 6-c and 6-d). Anti-FADD antibody was obtained from Santa Cruz Biotechnology and anti-Bid antibody was obtained from BD Pharmingen.

Based on the finding that caspase 8 was activated by FUT in a concentration-dependent manner, it was revealed that FUT plays an important role in initiating the apoptosis signaling cascade in addition to inhibition of NF-κB activation and induction of TNFRI expression. Moreover, as shown in Figures 6-b and 6-c, FUT treatment promoted FADD phosphorylation overtime and later on promoted caspase 8 activation. This indicates that FADD is involved in the caspase 8 activation-initiated apoptosis signaling cascade in the apoptosis pathway induced by TNF. The results also showed an overtime increase in the expression of JBid, the degradation product of Bid, which is essential for a JNK-mediated pathway (Figure 6-d). Taken together, it was revealed that two independent pathways: FADD-mediated and JNK-mediated were involved in caspase 8 activation by FUT.

### Example 8

### Synergistic effect of FUT and TNF-α: Part 1;

After the following treatment on the human pancreas cancer cell line MDAPanc-28, plasmatic compartment was prepared and Western blot was performed to examine the expression of phosphorylated FADD, caspase 8, NF-κB and IκBα. Cells which had been stimulated with TNF-α (5 ng/ml) for 24 hours after pretreatment with FUT (80 µg/ml) showed enhanced FADD phosphorylation and caspase 8 activation compared to cells treated with FUT alone as shown in Figure 7-a due to TNF-α treatment. It was also indicated that FUT treatment inhibits TNF-α-activated NF-κB (Figure 7-b). The same was true for IκBα expression (Figure 7-c).

### Example 9

### Synenrgistic effect of FUT and TNF-α: Part 2;

The present inventors examined the concomitant effect of FUT and TNF-α on apoptosis in cancer cells. After treatment with TNF-α for 24 hours, the human pancreas cancer cell line MDAPanc-28 was further cultured for 24 hours with various concentrations of FUT being added. Cells prepared were then subjected to an MTT assay according to the method generally known to calculate the cell survival rates. Also, DNA was extracted to perform a DNA fragmentation assay.

More specifically, with regard to the MTT assay, each well was seeded with 2000 cells per 100 µl of culture medium in a 96-well microtiterplate, followed by the above mentioned treatment. After plate centrifugation, each well received 5 mg/ml of MTT (Sigma) PBS solution instead of culture medium and was incubated for 4 hours at 37°C. After removing the supernatant, 100 µl of DMSO were added to dissolve the cells. The absorbance of the samples was measured at 570 nm by using a microplate reader. 8 wells were subjected to the same condition in this experiment. Two independent experiments were performed, and the average was calculated. The DNA fragmentation assay was performed by the method similar to Example 4.

The results are shown in Figure 8. Compared with control cells, the cell survival rate significantly declined in cells treated with FUT. Moreover, cells exhibited a declining trend in cell survival rate when treated with FUT in combination with TNF-α (Figure 8-a). Also, cells treated with 5 ng/ml of TNF-α alone showed no DNA fragmentation, whereas cell treated with TNF-α in combination with FUT did show DNA fragmentation (Figure 8-b). These results indicated that the administration of FUT in combination with TNF-α can synergetically induce apoptosis in cancer cells.

### Example 10

### Mechanism whereby FUT activates caspase 8;

In order to confirm whether FUT treatment-induced elevation in TNFRI expression is essential for caspase 8 activation, cells in which TNFRI expression was downregulated using siRNA (small interfering RNA) were prepared.

As shown in Figure 9, cells in which TNFRI expression was downregulated showed neither FADD phosphorylation nor caspase 8 activation even after FUT treatment. Therefore, it was revealed that FUT-induced activation of caspase 8 is TNFRI-dependent.

### Example 11

### Inhibition of the antitumor agent gemcitabine-induced activation of NF-κB by FUT treatment;

Drug resistance caused by some chemotherapeutic agents via induction of NF-κB activation within the cell has been posing a problem. Therefore, the present inventors first confirmed whether gemcitabine acts on the human pancreas cancer cell line MDAPanc-28 to induce NF-κB activation. After treatment with gemcitabine for 12 hours, the human pancreas cancer cell line MDAPanc-28 was subjected to a gel shift assay according to the method generally known and was examined for the presence of NF-κB activation. More specifically, the experiment was performed by the method similar to the method described in Example 1. As shown in Figure 10-a, the results confirmed that NF-κB activation was induced by gemcitabine treatment.

The present inventors then examined the concomitant effects of gemcitabine and FUT. At the same time, the present inventors also made a comparison with the other NF-κB inhibitors PS1145 and PS341. The human pancreas cancer cell line MDAPanc-28 was treated with gemcitabine for 3 hours after pretreatment with FUT, PS1145 or PS341 for 3 hours. Subsequently, a gel shift assay was performed to examine the effect on NF-κB activation. As shown in Figure 10-b, the results revealed that FUT had an inhibitory effect on gemcitabine-induced activation of NF-κB. Although the other two agents also showed a similar trend, the inhibitory effect of FUT was most significant.

### Example 12

### Effect of FUT treatment on the living cell ratio in the human pancreas cancer cell line MDAPanc-28;

FUT, PS1145 and PS341 all showed an inhibitory effect on NF-κB activation. However, the agents are of different types with different mechanisms of action. Therefore, the present inventors examined the effect of each agent on the living cell ratio in the human pancreas cancer cell line MDAPanc-28 singularly. After being treated with each agent for 24 hours and 48 hours, the human pancreas cancer cell line MDAPanc-28 was subjected to a MTT assay. The MTT assay was performed by the method described in Example 9. As shown in Figure 11-a, the results indicated that cell proliferation was inhibited in a FUT concentration-dependent manner in the human pancreas cancer cell line MDAPanc-28 and that the living cell ratio slightly increased at 48 hours compared to the ratio at 24 hours in cells treated with FUT. In contrast, PS1145 showed a concentration-dependent inhibitory effect on proliferation in cells after 48-hour treatment, whereas it did not show any significant inhibitory effect in cells after 24-hour treatment. Also, PS341 showed no significant inhibitory effect on proliferation in both cells after 24-hour treatment and 48-hour treatment (Figures 11-b and 11-c). Taken together, it was revealed that FUT and PS1145 had an inhibitory effect on proliferation in the human pancreas cancer cell line MDAPanc-28 and that FUT induced a more rapid inhibitory effect than PS1145.

### Example 13

### Examination of concomitant effect of gemcitabine and NF-κB on apoptosis in the human pancreas cancer cell line MDAPanc-28;

FUT, PS1145 and PS341 were revealed to have an inhibitory effect on gemcitabine-induced activation of NF-κB. The present inventors next examined the effect of these agents on apoptosis induction in the human pancreas cancer cell line MDAPanc-28. Apoptosis ratio was calculated for cells treated with gemcitabine for 48 hours after pretreatment with FUT, PS1145 or PS341 for 3 hours. Flow cytometry was used to confirm the occurrence of apoptosis. Cells were collected after treatments with the agents and fixed in 70% ethanol at -20°C for 24 hours. After rinsing with PBS, cells were suspended in PBS containing 50 µg/ml of propium iodide, 0.1% Triton X-100, 0.1% sodium citrate and 1 µg/ml of DNase free RNase. The samples were incubated at room temperature for 30 minutes and the amount of DNA was measured by using FACScan. As shown in Figure 12, the results indicated that gemcitabine administered in combination with FUT, PS1145 or PS341 further enhanced the effect on apoptosis induction, although gemcitabine alone induced apoptosis.

### Example 14

### Effect of FUT treatment on normal cells;

As an effective antitumor agent, it is important to demonstrate that it does not affect normal cells in order to be recognized as an effective antitumor agent. Therefore, the present inventors next examined the effect of FUT treatment on normal cells. Murine fiber cells were used as normal cells. The results indicated that FUT treatment at a concentration of 80 µg/ml had no effect on cell survival rate (Figure 13). In contrast, the other NF-κB inhibitors PS1145 and PS341 significantly decreased the survival rate of normal cells when used at concentrations under which apoptosis was induced in cells.

### Example 15

### Inhibition of MMP-2 and MMP-9 expression by FUT treatment;

Cytoplasmic fraction was prepared from the human pancreas cancer cell line MDAPanc-28 treated with either 0 µg/ml or 80 µg/ml of FUT. Western blot was performed for the obtained cytoplasmic proteins to examine MMP-2 and MMP-9 expression. More specifically, the experiment was performed by the method described in Example 2. Rabbit polyclonal anti-MMP-2 antibody, goat polyclonal anti-MMP-9 antibody and mouse anti-B-actin antibody (Santa Cruz Biotechnology, Inc.) were used for detection. As shown in Figure 14, the results indicated that MMP-2 and MMP-9 expression was inhibited by FUT in a concentration-dependent manner.

### Example 16

### Inhibition of infiltration of pancreas cancer cell line by FUT treatment;

A matrigel infiltration assay was performed to examine an inhibitory effect on infiltration in the human pancreas cancer cell line MDAPanc-28 by FUT treatment. Cells treated with 80 µg/ml of FUT for 24 hours and untreated cells were respectively inoculated to a matrigel-coated chamber at a concentration of 20000 cells per well for a 22-hour incubation at 37°C either in the presence or absence of 80 µg/ml of FUT. The numbers of infiltrated cells were then counted under the microscope after Wright-Giemsa staining according to the method generally known. The results are shown in Figure 15. It was indicated that cells pretreated with FUT showed a significant inhibitory effect on infiltration.

### Example 17

### Effect of FUT175 and gemcitabine administration in vivo;

Male nude mice (8 weeks of age) were subcutaneously transplanted with the human pancreas cancer cell line Panc-1 (5 × 106 cells), and 6 weeks after transplantation the mice were divided into 3 groups so that each group had a similar average tumor radius. Each group was then subjected to the following treatment for 6 weeks respectively: the control group received no treatment (n=4); the GEM group (gemcitabine administered group) was administered with gemcitabine (100 mg/kg, once a week, i.p.) (n=4); and the FUT group was administered with FUT-175 (30 mg/kg, three times a week, i.p.) + gemcitabine (100 mg/kg, once a week, i.p.) (n=4). Tumor volume, the body weight and extirpated tumor weight were measured and pictures were taken for each group of mice.

Figure 16 shows the results of measuring tumor volume. Tumor volume was reduced to approximately 50% level of the control in the gemcitabine-alone administered group and was reduced to approximately 25% level of the control in the group administered with gemcitabine in combination with FUT. This was a surprising result which no one ever expected. Figure 17 shows the results of measuring body weight of mice after administration. In contrast to the gemcitabine-alone group, which showed weight reduction, the group administered with gemcitabine in combination with FUT surprisingly showed no weight reduction, similarly to the control group. Extirpated tumor weight was also reduced to approximately 50% level of the control in the gemcitabine-alone group and was reduced to approximately 6% level of the control in the group administered with gemcitabine in combination with FUT as shown in Figure 18. Figure 19 shows a set of pictures of mice taken after the treatments, showing the tumors were significantly reduced in the group administered with gemcitabine in combination with FUT.

Based on the results obtained from the above described experiments, the administration of gemcitabine in combination with FUT not only exerted a significant antitumor effect but also caused no serious adverse effects such as weight reduction. This will bring tremendously good news for tumor patients and the coadministration is hoped to be put into practice for clinical use in the near future.

### Industrial Applicability

Antitumor agents of the present invention are novel agents which inhibit tumor cell proliferation through a mechanism whereby the agents inhibit NF-κB activation via inhibition on the phosphorylation of Iκ-Bα by inhibiting IKK in tumor cells. The agents are particularly effective for pancreas cancer, for which no effective therapies or chemotherapeutic agents are available at present. Moreover, the agents have an inhibitory effect on cancer metastasis. Furthermore, the agents have an inhibitory effect on drug resistance caused by existing antitumor agents. Therefore, the antitumor agents of the present invention do not only exert an antitumor effect when administered alone but also they exert a further enhanced antitumor effect when administered in combination with the existing antitumor agents. What is most remarkable is that the significant antitumor effects of the same have been confirmed in an animal study. The safety of the same has already been confirmed as the agents have already been approved. Therefore, the agents are expected to be put into practical use as safe antitumor agents with no possibility of causing adverse effects. In particular, administration of gemcitabine hydrochloride in combination with FUT showed no adverse effects such as weight reduction and therefore is highly promising due to its efficacy in tumor therapy and its safety.

## Claims

1. An antitumor agent, a cancer metastasis inhibitor, or a cancer infiltration inhibitor having as an active ingredient 6'-amidino-2'-naphthyl 4-guanidinobenzoate represented by the following formula or a pharmacologically acceptable salt thereof.

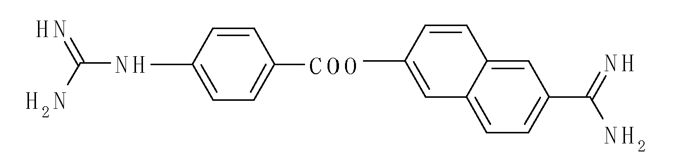

2. The antitumor agent, the cancer metastasis inhibitor, or the cancer infiltration inhibitor according to claim 1, wherein the salt is mesylate.

3. The antitumor agent, the cancer metastasis inhibitor, or the cancer infiltration inhibitor according to either claim 1 or 2, wherein the agent or inhibitor is an antitumor agent, a cancer metastasis inhibitor, or a cancer infiltration inhibitor for pancreas cancer respectively.

4. An NF-κB inhibitor having as an active ingredient 6'-amidino-2'-naphthyl 4-guanidinobenzoate or a pharmacologically acceptable salt thereof.

5. The NF-κB inhibitor according to claim 4, wherein the salt is mesylate.

6. An IKK (IκB phosphorylating enzyme) inhibitor having as an active ingredient 6'-amidino-2'-naphthyl 4-guanidinobenzoate or a pharmacologically acceptable salt thereof.

7. The IKK (IκB phosphorylating enzyme) inhibitor according to claim 6, wherein the salt is mesylate.

8. A medicine comprising the agent according to any one of claims 1 to 7 in combination with other antitumor agents.

9. The medicine according to claim 8, wherein the other antitumor agents are one or more antitumor agents selected from the group consisting of alkylating agents, antimetabolites, antibiotics, plant alkaloids, platinum complex derivatives and hormones.

10. The medicine according to either claim 8 or 9, wherein the other antitumor agents are one or more antitumor agents selected from the group consisting of gemcitabine hydrochloride, nitrogen mustard N-oxide, cyclophosphamide, melphalan, carboquone, busulfan, nimastine hydrochloride, ranimastine, dacarbazine, fluorouracil, tegafur, cytarabine, ansaitabine hydrochloride, broxyuridine, doxifluuridine, mercaptopurine, thioinosin, methotrexate, mitomycin, bleomycin, daunorubicin hydrochloride, doxorubicin hydrochloride, pirarubicin hydrochloride, aclarubicin hydrochloride, neocarzinostatin, actinomycin D, vincristin hydrosulfate, vindesin hydrosulfate, vinblastin hydrosulfate, etoposide, tamoxifen citrate, procarbazine hydrochloride, mitobronitol, mitoxanthone hydrochloride, carboplatin and cisplatin.

11. A method for treating or preventing cancers or tumors, **characterized in that** the method comprises administering the antitumor agent, the cancer metastasis inhibitor, or the cancer infiltration inhibitor according to claim 1 to a cancer patient or tumor patient.

12. The method for treating or preventing cancers or tumors according to claim 11, **characterized in that** the method comprises administering the antitumor agent, the cancer metastasis inhibitor, or the cancer infiltration inhibitor according to claim 1 in combination with other antitumor agents simultaneously or separately at any interval to a cancer patient or tumor patient.


SEQUENCE LISTING

<110>  Torii Pharmaceutical Co.

<120>  anti-tumor effects of FUT

<130>  TR0505J1NF

<140>  TR0505J1NF
<141>  2005-05-31

<160>  9

<170>  PatentIn version 3.1

<210>  1
<211>  22
<212>  DNA
<213>  Artificial

<220>
<223>  Oligonucleotide primer used in PCR

<400>  1
agttgagggg actttcccag gc      22


<210>  2
<211>  21
<212>  DNA
<213>  Artificial

<220>
<223>  Oligonucleotide primer used in PCR

<400>  2
agcgggacag gaagagctgg a         21


<210>  3
<211>  21
<212>  DNA
<213>  Artificial

<220>
<223> Oligonucleotide primer used in PCR

<400> 3
agcgggacag ctcgagctgg a 21

<210> 4
<211> 21
<212> DNA
<213> Artificial

<220>
<223> Oligonucleotide primer used in PCR

<400> 4
tgtcgaatgc aaatcactag a 21

<210> 5
<211> 20
<212> DNA
<213> Artificial

<220>
<223> Oligonucleotide primer used in PCR

<400> 5
actaatctgc gccaccttct 20

<210> 6
<211> 20
<212> DNA
<213> Artificial

<220>
<223> Oligonucleotide primer used in PCR

<400> 6
atgaaccacg attggggagc 20

<210> 7
<211> 20
<212> DNA

<213>  Artificial

<220>
<223>  Oligonucleotide primer used in PCR

<400>  7
agatctggga ctcctgtgct                    20


<210>  8
<211>  20
<212>  DNA
<213>  Artificial

<220>
<223>  Oligonucleotide primer used in PCR

<400>  8
gttaaagaac gttgggcctc                    20


<210>  9
<211>  20
<212>  DNA
<213>  Artificial

<220>
<223>  Oligonucleotide primer used in PCR

<400>  9
tcctcctcca gctcttcctg                    20